(19) Europäisches Patentamt
European Patent Office
Office européen des brevets

(11) Veröffentlichungsnummer: **0 015 388**
**B1**

(12) # EUROPÄISCHE PATENTSCHRIFT

(45) Veröffentlichungstag der Patentschrift: **21.10.81**

(51) Int. Cl.³: **C 12 P 17/12**

(21) Anmeldenummer: **80100552.1**

(22) Anmeldetag: **04.02.80**

(54) **1-Desoxynojirimycin-Herstellung.**

(30) Priorität: **23.02.79 DE 2907190**

(43) Veröffentlichungstag der Anmeldung: **17.09.80 (Patentblatt 80/19)**

(45) Bekanntmachung des Hinweises auf die Patenterteilung: **21.10.81 Patentblatt 81/42**

(84) Benannte Vertragsstaaten: **AT BE CH DE FR GB IT NL SE**

(56) Entgegenhaltungen:
**FR - A - 2 378 854**
**FR - A - 2 385 733**

(73) Patentinhaber: **BAYER AG**
**Zentralbereich Patente, Marken und Lizenzen**
**D-5090 Leverkusen 1, Bayerwerk (DE)**

(72) Erfinder: **Frommer, Werner, Dr.**
**Claudiusweg 17**
**D-5600 Wuppertal-1 (DE)**
Erfinder: **Schmidt, Delf, Dr.**
**Am Eckbusch 55b**
**D-5600 Wuppertal-1 (DE)**

**EP 0 015 388 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

1-Desoxynojirimycin-Herstellung

Es ist bekannt, daß eine Reihe von Actinomyceten, vor allem Actinoplanaceen Inhibitoren für Glykosidhydrolasen, vorzugsweise kohlenhydratspaltender Enzyme des Verdauungstraktes bilden (DE-A 2 064 092).

Weiterhin weiß man, daß Nojirimycin, ein bakteriostatisch wirkendes Antibioticum aus Stämmen der Gattung Streptomyces, gewisse mikrobielle α-Glucosidasen hemmt (T. NIWA et al. Agr. Biol. Chem. *34*, 966 (1970)).

Ferner ist bekannt, daß Inhibitoren für Glykosidhydrolasen, insbesondere 1-Desoxynojirimycin, von Organismen der Familie Bacillaceae, besonders von Stämmen der Gattung Bacillus, in üblichen Nährlösungen bei Temperaturen von etwa 15 bis etwa 80 °C unter Belüftung in üblichen Fermentationsgefäßen gebildet werden, wobei die Kultivierung innerhalb des angegebenen Temperaturbereiches stets bei einer konstanten Temperatur durchgeführt wird (DE-A 27 26 899).

Die Übertragung dieses im Labormaßstab sehr guten Verfahrens in einen Großfermenter war bisher von Ausbeuteverlusten begleitet. Dieser Nachteil konnte jetzt überraschenderweise behoben werden, gleichzeitig wurde durch Veränderung einiger Verfahrensparameter eine Ausbeutesteigerung gegenüber dem bisherigen Labor-Verfahren um ein mehrfaches erreicht.

Die vorliegende Erfindung betrifft daher ein verbessertes Verfahren zur Herstellung von 1-Desoxynojirimycin durch Kultivierung von Organismen der Familie Bacillaceae in üblichen Nährlösungen bei Temperaturen von etwa 15 bis 80 °C unter Belüftung in üblichen Fermentationsgefäßen und anschließende Isolierung des 1-Desoxynojirimycins, das dadurch gekennzeichnet ist, daß man in den Nährlösungen als Kohlenstoffquelle Sorbit einsetzt. Vorzugsweise ist das Verfahren ferner dadurch gekennzeichnet, daß man die Kultivierung bei einer Temperatur zwischen 15 und 30 °C startet und in einem Zeitraum, der die zweite Hälfte der logarithmischen Wachstumsphase und die erste Hälfte der stationären Phase umfaßt, die Temperatur auf 30 bis 50 °C erhöht. Ganz besonders bevorzugt wird das Verfahren ferner so ausgeführt, daß man während der gegebenenfalls stufenweisen Vermehrung des Impfgutes das Impfgut wenigstens einmal auf eine Temperatur von 80 bis 100 °C erhitzt und wieder auf Fermentationstemperatur abkühlt.

Besonders reines 1-Desoxynojirimycin in sehr guter Ausbeute wird dadurch gewonnen, daß man die Isolierung in folgender Weise durchführt. Nach der Abtrennung von den Zellen wird die Kulturlösung zunächst an stark sauren Ionenaustauschern ($H^+$-Form) adsorbiert, mit 0,5 bis 2 N Ammoniaklösung desorbiert, die aktiven Fraktionen an einem schwach sauren Ionenaustauscher ($H^+$-Form) adsorbiert, mit 0,02 bis 0,1 N Mineralsäuren, z.B. Salzsäure, desorbiert, die aktiven Fraktionen über einen stark basischen Ionenaustauscher ($OH^-$-Form) chromatographiert, das basische aktive Eluat zur Trockene eingedampft, bei erhöhter Temperatur in einem polaren Lösungsmittel, vorzugsweise 60 bis 100 %igem wäßrigem Methanol aufgenommen und durch Abkühlung kristallisiert.

Vorzugsweise verwendet man Organismen der Gattung Bacillus, wobei die Arten B. subtilis, B. subtilis var. niger, B. amyloliquefaciens, B. coagulans, B. longisporus und B. polymyxa besonders geeignet sind. Von diesen Arten sind die Stämme DSM 292, DSM 356, DSM 36, DSM 740, DSM 741, DSM 1, DSM 479, DSM 365, DSM 742, DSM 7, DSM 372, DSM 675, DSM 704, DSM 1 060, DSM 1 061, DSM 1 062, DSM 1 063, DSM 1 064, DSM 1 065, DSM 1 066 und DSM 1 076 bevorzugt.

Die besten Ergebnisse werden mit den Stämmen DSM 7 und DSM 704 erzielt.

Die Stämme sind bei der deutschen Sammlung für Mikroorganismen, Göttingen, hinterlegt und von dort zu beziehen. Ausführliche Beschreibungen der Stämme, soweit sie als Patentstämme beider DSM hinterlegt sind, sind der DE-A 27 26 899 zu entnehmen. Diese Literaturstelle beschreibt auch ausführlich Methoden zur Auffindung geeigneter Stämme die technische Verwendung von 1-Desoxynojirimycin als Inhibitor für Glykosidhydrolasen, Testmethoden zur Feststellung der Inhibitionswirkung des 1-Desoxynojirimycins als Amylase-Inhibitor, Saccharase-Inhibitor und Maltase-Inhibitor und pharmazeutische Zubereitungen von 1-Desoxynojirimycin und deren Herstellung.

Als Stickstoffquelle für die Nährlösungen kommen die verschiedenartigsten organischen Substanzen infrage, beispielsweise Hefeextrakt, Sojamehl, Peptone und Fleischextrakt.

Die Konzentration des Sorbits und der Stickstoffquellen sowie der Nährsalze, von denen $FeSO_4$, $CaCO_3$ und $MgSO_4$ beispielhaft erwähnt seien, können in weiten Grenzen schwanken. Auf den gesonderten Zusatz von Nährsalzen kann in manchen Fällen ganz verzichtet werden, da sie oftmals in den komplexen Stickstoffquellen als Beimengungen enthalten sind.

Da die Bildung von 1-Desoxynojirimycin oft stark von der Zusammensetzung der Nährböden abhängig ist, empfiehlt es sich, die Stämme zur Optimierung der Produktionsleistung in verschiedenen Nährlösungen zu kultivieren. Entsprechende Vorschläge können den Beispielen entnommen werden.

1-Desoxynojirimycin ist ein kristallines Produkt mit 540 000 SIE/g (SIE = Saccharase-Inhibitor-Einheit, bezüglich der Definition wird auf die CE-A 27 26 899, Seite 4, verwiesen).

Die Verbindung hat die Strukturformel

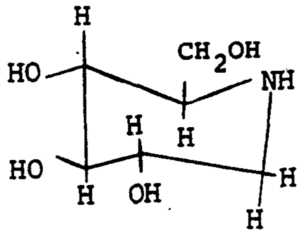

Die Verbindung wurde erstmals von S. Inoye et al. (Tetrahedron *23* 2125 (1968)) beschrieben. Die Autoren stellten 1-Desoxynojirimycin auf chemischem Wege durch Hydrieren des Antibioticums Nojirimycin her. Das Ausgangsprodukt Nojirimycin wird nach T. Niida et al. (J. Antibiotics, Ser. A. 20, 62 (1967)) durch Fermentation von Organismen der Gattung streptomyces erhalten.

1-Desoxynojirimycin dient weiterhin als Ausgangsprodukt zur Herstellung N-substituierter 1-Desoxynojirimycine, die ebenfalls inhibitorische Wirkung für Glycosidhydrolasen aufweisen (DE-A 27 38 717).

Beispiel 1

Beimpft man einen 1 l Erlenmeyerkolben, der 120 ml einer Nährlösung der Zusammensetzung
6,5 Gew.-% Glycerin
3,0 Gew.-% Sojamehl
0,2 Gew.-% $CaCO_3$
Leitungswasser bis 100 Gew.-%
enthält und durch 30-minütiges Erhitzen auf 121 °C sterilisiert wurde, mit einer Sporensuspension des Stammes DSM 7 und bebrütet man den Kolben auf einer Rundschüttelmaschine bei 28 °C, so erhält man nach 5-tägiger Bebrütung eine Aktivität von 630 SIE/ml.

Beispiel 2

Beimpft man einen 1 l Erlenmeyerkolben, der 120 ml einer Nährlösung der Zusammensetzung
6,5 Gew.-% Sorbit
3,0 Gew.-% Sojamehl
0,2 Gew.-% $CaCO_3$
Leitungswasser bis 100 Gew.-%
enthält und durch 30-minütiges Erhitzen auf 121 °C sterilisiert wurde, mit einer Sporensuspension des Stammes DSM 7 und bebrütet man den Kolben auf einer Rundschüttelmaschine bei 28 °C, so erhält man nach 5-tägiger Bebrütung eine Aktivität von 1 320 SIE/ml.

Beispiel 3

Bebrütet man einen Ansatz nach Beispiel 2 45 Stunden bei 28 °C und erhöht dann die Temperatur auf 35 °C, so erhält man nach insgesamt 5-tägiger Fermentation eine Kulturbrühe mit 1 820 SIE/ml.

Beispiel 4

Bebrütet man einen Ansatz nach Beispiel 2 65 Stunden bei 28 °C und erhöht dann die Temperatur auf 42 °C, so erhält man nach insgesamt 5-tägiger Fermentation eine Kulturbrühe mit 2 070 SIE/ml.

Beispiel 5

Bebrütet man einen Ansatz nach Beispiel 2 45 Stunden bei 28 °C und erhöht dann die Temperatur auf 42 °C, so erhält man nach insgesamt 5-tägiger Fermentation eine Kulturbrühe mit 2 530 SIE/ml.

Beispiel 6

Beimpft man einen 1 l Erlenmeyerkolben, der 120 ml einer Nährlösung der Zusammensetzung
5,0 Gew.-% Glycerin
0,9 Gew.-% Kaseinhydrolysat
1,0 Gew.-% Hefeextrakt
0,1 Gew.-% $CaCO_3$
0,1 Gew.-% $K_2HPO_4$
Leitungswasser bis 100 Gew.-%
enthält und durch 30-minütiges Erhitzen auf 121 °C sterilisiert wurde, mit einer Sporensuspension des Stammes DSM 704 und bebrütet den Kolben auf einer Rundschüttelmaschine bei 28 °C, so erhält man nach 5-tägiger Bebrütung eine Aktivität von 25 SIE/ml.

Beispiel 7

Beimpft man einen 1 l Erlenmeyerkolben, der 120 ml einer Nährlösung der Zusammensetzung
5,0 Gew.-% Sorbit
0,9 Gew.-% Kaseinhydrolysat
1,0 Gew.-% Hefeextrakt
0,1 Gew.-% $CaCO_3$
0,1 Gew.-% $K_2HPO_4$
Leitungswasser bis 100 Gew.-%
enthält und durch 30-minütiges Erhitzen auf 121 °C sterilisiert wurde, mit einer Sporensuspension des Stammes DSM 704 und bebrütet man den Kolben auf einer Rundschüttelmaschine bei 28 °C, so erhält man nach 5-tägiger Bebrütung eine Aktivität von 120 SIE/ml.

Beispiel 8

Bebrütet man einen Ansatz nach Beispiel 7 45 Stunden bei 28 °C und erhöht dann die Temperatur auf 35 °C, so erhält man nach insgesamt 5-tägiger Bebrütung eine Kulturbrühe mit 339 SIE/ml.

Beispiel 9

Bebrütet man einen Ansatz nach Beispiel 7 45 Stunden bei 28 °C und erhöht dann die Temperatur auf 42 °C, so erhält man nach insbesamt 5-tägiger Bebrütung eine Kulturbrühe mit 342 SIE/ml.

Beispiel 10

Beimpft man mit 1 ml aus einem Ansatz nach Beispiel 1 erneut einen Schüttelkolben mit derselben Nährlösung und wiederholt dieses Vorgehen mehrfach, so wie es notwendig ist, wenn man größere Fermenter ansetzt, so vermindert sich die Ausbeute an SIE/ml von Passage zu Passage. Man kann diesen Abfall in der Aktivität vermindern, wenn man nach Ende einer Passage die Kultur kurz aufkocht.

| Anzahl der Passagen | Kurzzeiterhitzung am Ende der X. Passage | Endtiter 4. Passage SIE/ml |
|---|---|---|
| 4 | — | 293 |
| 4 | 3. | 413 |
| 4 | 1.2.3. | 662 |

Beispiel 11

Führt man den Stamm DSM 704 in eine Nährlösung der Zusammensetzung
7,5 Gew.-% Malzextrakt
0,3 Gew.-% Kaseinhydrolysat
0,7 Gew.-% Hefeextrakt
0,3 Gew.-% $CaCO_3$
0,3 % $K_2HPO_4$
Leitungswasser bis 100 Gew.-%
die durch 30-minütiges Erhitzen auf 121 °C sterilisiert und danach mit $K_2CO_3$ auf pH 6,6 bis 6,8 eingestellt wurde, wie in Beispiel 6 über mehrere Passagen. So verminderst sich die Ausbeute von Passage zu Passage. Man kann diesen Abfall vermindern, wenn man nach Ende jeder Passage die Kultur kurz aufkocht.

| Anzahl der Passagen | Ausbeute in SIE/ml nach 6-tägiger Fermentation | |
|---|---|---|
| | ohne Aufkochen | mit Aufkochen |
| 1. | 160 | 150 |
| 3. | 145 | 138 |
| 5. | < 58 | 116 |
| 6. | < 41 | 135 |

Beispiel 12

360 l Kulturbrühe nach Beispiel 2 wurden mit 3,6 l 2 m Oxalsäure versetzt und anschließend mit halbkonzentiertem $HNO_3$ auf pH 3,2 eingestellt. Man rührte 30 Minuten und zentrifugierte anschließend in einer Überlaufzentrifuge bei 2 500 Upm. Die 300 l leicht trüben Überstandes wurden mit einer Fließrate von 200 l/h über eine 30 × 50 cm mit dem stark sauren Austauscher Lewatit ® SC 104 $H^+$ (Bayer AG) gefüllte Säule gegeben.

Anschließend wurde mit 400 l entionisiertem Wasser gewaschen. Durchlauf und Waschwasser waren inaktiv und wurden verworfen. Die Säule wurde anschließend mit 130 l 1 n Ammoniak desorbiert, das Eluat wurde in 10 l-Fraktionen aufgefangen. Die aktiven Fraktionen 4 bis 9 wurden vereinigt und über eine 15 × 80 cm mit dem schwach sauren Austaucher Lewatit ® CNP $H^+$ (Bayer AG) gefüllte Säule gegeben. Man wusch mit 150 l entionisiertem Wasser und verwarf die inaktiven Durchlauf- und Waschwasserfraktionen. Anschließend wurde mit einer Fließrate von 20 l/h mit 0,05 n HCl eluiert. Das Eluat wurde in 10 l-Fraktionen aufgefangen, die aktiven Fraktionen 3 bis 10 vereinigt und über eine 15 × 40 cm mit dem stark basischen Harz Lewatit ® M 500 $OH^-$ (Bayer AG) gefüllte Säule gegeben. Man eluierte mit destilliertem Wasser nach. Durchlauf und Waschwasser der M 500-Säule wurden in 10 l-Portionen fraktioniert, die aktiven Fraktionen 1 bis 12 vereinigt und im Vakuum auf ca. 400 ml eingeengt. Das erhaltene sirupöse Konzentrat versetzte man in der Wärme (50 bis 60 °C) mit 1 600 ml Methanol, filtrierte heiß von ungelösten Anteilen ab und ließ das klare Filtrat erkalten. Über Nacht setzte nach Zugabe von Impfkristallen die Kristallisation ein. Nach 2 Tagen bei 4 °C wurden die Kristalle abgesaugt, 2 mal mit Methanol, 1 mal mit Aceton und 1 mal mit Ether gewaschen und im Vakuum getrocknet. Ausbeute 360 g reine Desoxynojirimycinbase. Weitere Rohsubstanz kann aus den Kristallisationsmutterlaugen durch weiteres Einengen erhalten werden.

Beispiel 13

Die Bindung von Desoxynojirimycin an stark saure Austauscher in der $H^+$-Form kann auch im batch erfolgen, jedoch muß dann — da bei pH-Werten β die Desorption des Desoxynojirimycins beginnt — ein stark oder schwach basischer Anionenaustauscher zur Bindung der bei der Adsorption der Substanz freigesetzten $H^+$-Ionen zugesetzt werden.

Zu 60 l Fermentationsansatz (pH 8,0) nach Beispiel 1 setzte man ohne vorherige Abtrennung der Zellen 7,2 kg Lewatit ® SC 104 $H^+$ und 2,4 kg Lewatit ® MP 62 $OH^-$ hinzu. Man rührte 30 Minuten und trennte anschließend in einer Siebschneckenschleuder ab. Das Filtrat mit Zellen (pH 3,2) war inaktiv und wurde verworfen. Das Harz wurde mit destilliertem Wasser gewaschen und anschließend mehrmals flotiert. Das leichtere Lewatit ® MP 62 Harz wurde abgeschöpft, das am Boden verbleibende desoxynojirimycinbeladene Lewatit ® SC 104 Harz wurde in eine loxloo cm Säule überführt und anschließend mit 1 n $NH_3$, wie in Beispiel 8 beschrieben, eluiert. Die weitere Reinigung über Lewatit ® CNP $H^+$, Lewatit ® M 500 $OH^+$ und anschließende Kristallisation wurde, wie in Beispiel 8 beschrieben, durchgeführt. Ausbeute 31 g reine Desoxynojirimycin-base. Auf die oben beschriebene Zerlegung des

Mischbettharzes durch Flotation vor Elution mit Ammoniak kann gegebenenfalls verzichtet werden, ohne daß sich an der Reinheit des Endproduktes etwas ändert.

**Ansprüche**

1. Verfahren zur Herstellung von 1-Desoxynojirimycin durch Kultivierung von Stämmen der Familie Bacillaceae in Nährlösungen bei Temperaturen von 15 bis etwa 80 °C unter Belüftung in üblichen Fermentationsgefäßen und anschließende Isolierung, dadurch gekennzeichnet, daß man in der Nährlösung als Kohlenstoffquelle Sorbit einsetzt.

2. Verfahren nach Anspruch 1, dadurch gekennzeichnet, daß man die Kultivierung bei einer Temperatur zwischen 15 und 30 °C startet und in einem Zeitraum, der die zweite Hälfte der logarithmischen Wachstumsphase und die erste Hälfte der stationären Phase umfaßt, die Temperatur auf 30 bis 50 °C erhöht.

3. Verfahren nach Anspruch 1, dadurch gekennzeichnet, daß man während der gegebenenfalls stufenweisen Vermehrung des Impfgutes das Impfgut wenigstens einmal auf eine Temperatur von 80 bis 100 °C erhitzt und wieder auf Fermentationstemperatur abkühlt.

4. Verfahren nach Anspruch 1, dadurch gekennzeichnet, daß man 1-Desoxynojirimycin direkt aus der Fermentationslösung oder — nach vorheriger Abtrennung der Zellen — aus dem Kulturfiltrat an stark saure Ionenaustauscher ($H^+$-Form) adsorbiert, mit 0,5 bis 2 N Ammoniaklösung desorbiert, die aktiven Fraktionen an einem schwach sauren Ionenaustauscher ($H^+$-Vorm) adsorbiert, mit 0,02 bis 0,1 N-Salzsäure desorbiert, die aktiven Fraktionen über einen stark basischen Ionenaustauscher ($OH^-$-Form) chromatographiert, das aktive basische Eluat zur Trockene eindampft, bei erhöhter Temperatur in einem polaren Lösungsmittel aufnimmt und durch Abkühlung kristallisiert.

5. Verfahren nach Anspruch 1, dadurch gekennzeichnet, daß man Organismen der Gattung Bacillus verwendet.

6. Verfahren nach Anspruch 1, dadurch gekennzeichnet, daß man die Stämme DSM 7 oder DSM 704 verwendet.

**Claims**

1. A process for the production of 1-desoxynojirimicin by culturing strains of the Bacillaceae family in nutrient solutions at temperatures of 15 to about 80 °C in customary fermentation vessels, whilst aerating, and then isolating the product, characterised in that sorbitol is employed as the source of carbon in the nutrient solution.

.2. A process according to claim 1, characterised in that culturing is started at a temperature between 15 and 30 °C and the temperature is increased to 30 to 50 °C over a period which covers the second half of the logarithmic growth phase and the first half of the stationary phase.

3. A process according to claim 1, characterised in that during the optionally stepwise multiplication of the inoculum, the inoculum is heated to a temperature of 80 to 100 °C and cooled again to the fermentation temperature at least once.

4. A process according to claim 1, characterised in that 1-desoxynojirimicin is adsorbed onto strongly acid ion exchangers ($H^+$ form) directly from the fermentation solution or, after first separating off the cells, from the culture filtrate, and is desorbed with 0.5 to 2 N ammonia solution, the active fractions are adsorbed onto a weakly acid ion exchanger ($H^+$ form) and desorbed with 0.02 to 0.1 N hydrochloric acid, the active fractions are chromatographed over a strongly basic ion exchanger ($OH^-$ form), the active basic eluate is evaporated to dryness, the residue is taken up in a polar solvent at elevated temperature and the product is crystallised by cooling.

5. A process according to claim 1, characterised in that organisms of the genus Bacillus are used.

6. A process according to claim 1, characterised in that the strains DSM 7 or DSM 704 are used.

**Revendications**

1. Procédé de production de 1-désoxynojirimycine par culture de souches de la famille des Bacillaceae dans des solutions nutritives à des températures de 15 à environ 80 °C sous aération dans des appareils classiques de fermentation et isolement subséquent, caractérisé en ce qu'on utilise le sorbitol comme source de carbone dans la solution nutritive.

2. Procédé suivant la revendication 1, caractérisé en ce que la culture est déclenchée à une température comprise entre 15 et 30 °C et la température est élevée à une valeur de 30 à 50 °C en une période qui comprend la seconde moitié de la phase de croissance logarithmique et la première moitié de la phase stationnaire.

3. Procédé suivant la revendication 1, caractérisé en ce qu'on chauffe l'inoculum au moins une fois à une température de 80 à 100 °C et on le refroidit à nouveau à la température de fermentation pendant sa multiplication éventuellement graduelle.

4. Procédé suivant la revendication 1, caractérisé en ce qu'on fait adsorber de la 1-désoxynojirimycine directement à partir de la solution de fermentation ou — après séparation préalable des cellules — à partir du filtrat de culture sur des échangeurs ioniques fortement acides (forme $H^+$), on la désorbe avec une solution d'ammoniac 0,5 à 2 N, on adsorbe les fractions actives sur un échangeur ionique faiblement acide (forme $H^+$), on les désorbe avec de l'acide chlorhydrique 0,02 à 0,1 N, on chromatographie

les fractions actives sur un échangeur ionique fortement basique (forme $OH^-$), on concentre l'éluat basique actif par évaporation à sec, on le reprend à température élevée dans un solvant polaire et on le fait cristalliser par refroidissement.

5. Procédé suivant la revendication 1, caractérisé en ce qu'on utilise des organismes du genre Bacillus.

6. Procédé suivant la revendication 1, caractérisé en ce qu'on utilise les souches DSM 7 ou DSM 704.